# EUROPEAN PATENT SPECIFICATION

(11) **EP 0 667 961 B1**
(45) Date of publication and mention of the grant of the patent: **06.02.2002**
(21) Application number: 93924720.1
(22) Date of filing: 08.11.1993
(51) Int. Cl.: G01N 33/80, G01N 33/543

(54) **DETECTION OF ANTI-HPA ANTIBODIES**
NACHWEISS VON ANTI-HPA ANTIKÖRPERN
DETECTION D'ANTICORPS DIRIGES CONTRE L'ANTI-ANTIGENE PLAQUETTAIRE HUMAIN (HPA)

(30) Priority: 07.11.1992 GB 9223390
(43) Date of publication of application: 23.08.1995
(73) Proprietor: COMMON SERVICES AGENCY, Edinburgh EH5 3SE (GB)
(72) Inventor: BESSOS, Hagop, Edinburgh EH9 1HU (GB); MURPHY, William, Gerrard, Clanskeagh Dublin (IE)
(74) Representative: Horner, Martin Grenville
(86) International application number: GB9302297
(87) International publication number: WO9411740

(56) References cited:
- WO-A-89/02079
- JOURNAL OF BIOLOGICAL CHEMISTRY vol. 265, no. 30 , 25 October 1990 , BALTIMORE, MD US pages 18525 - 18530 D. KIRCHHOFER ET AL. 'Cation-dependent Changes in the Binding Specificity of the Platelet Receptor GPIIb/IIIa' cited in the application
- HAEMATOLOGICA vol. 76, no. 1 , January 1991 , ROMA, IT pages 20 - 27 PIERO BORZINI 'ELISA Procedures for the Characterization of Platelet Specific Antibodies: Evaluation of Some Variables' cited in the application
- TRANSFUSION MEDICINE vol. 2 , 1992 pages 181 - 188 V.KIEFEL 'The MAIPA Assay and its applications in immunohaematology' cited in the application
- CHEMICAL ABSTRACTS, vol. 95, no. 7, 17 August 1981, Columbus, Ohio, US; abstract no. 59684r, NEWMAN, PETER J. ET AL. 'Detection and Characterization of monoclonal antibodies to platelet membrane proteins' page 508 ; & J. CELL BIOL. vol. 90, no. 1 , 1981 pages 249 - 253
- BRITISH JOURNAL OF HAEMATOLOGY vol. 39, no. 2 , June 1978 , OXFORD, GB pages 195 - 207 A.E.G.KR. VON DEM BORNE ET AL. 'A Simple Immunofluorescence Test for the Detection of Platelet Antibodies' cited in the application
- THROMBOSIS RESEARCH vol. 59, no. 3 , 1 August 1990 , NEW YORK, NY, US pages 497 - 507 HAGOP BESSOS AND WILLIAM G. MURPHY 'A New Competitive Binding Enzyme-Linked Immunosorbent Assay for Glycocalicin in Plasma and Platelet Concentrate Supernatants' cited in the application
- JOURNAL OF IMMUNOLOGICAL METHODS. vol. 158, no. 2 , 3 February 1993 , NEW YORK US pages 197 - 200 CORNELIUS LÖLIGER, ELKE RÜHLMANN AND PETER KÜHNL 'A rapid and sensitive immunoassay for antibodies against alloantigens on human platelet glycoprotrins (BIPA)'
- THROMBOSIS AND HAEMOSTASIS vol. 66, no. 1 , 12 July 1991 , STUTTGART, DE pages 111 - 118 PETER J. NEWMAN 'Platelet GPIIb-IIIa: Molecular Variations and Alloantigens' cited in the application
- AMERICAN JOURNAL OF HEMATOLOGY vol. 38, no. 4 , December 1991 , NEW YORK, NY, US pages 314 - 320 J. FREEDMAN AND A. HORNSTEIN 'Simple Method for Differentiating Between HLA and Platelet-Specific Antibodies by Flow Cytometry' cited in the application

## Description

### TECHNICAL FIELD

The present invention relates to an immunoassay process and system for the immunological detection of antibodies to human platelet antigen 1 or 3 (HPA1 or HPA3) according to specific genotypes, and in particular to an enzyme linked immunoassay (ELISA) for their detection.

### BACKGROUND

HPA1 is a polymorphic determinant on the platelet membrane glycoprotein (GP) IIb/IIIa complex situated at the N-terminal region of GPIIIa. The complex consists of two non-covalently associated subunits GPIIb and GPIIIa. Divalent cations such as Ca²⁺ and Mg²⁺ are required for subunit association. The immunogenic property of HPA1 results in a variety of immune-mediated thrombocytopenias including post-transfusion purpura (PTP) and neonatal alloimmune thrombocytopenia (NAITP) (references 1, 2). HPA1 may exist as types HPA1a and HPA1b. Approximately 72% of the caucasian population is homozygous for HPA1a (platelet antigen 1 i.e. PLA1), 2% for HPA1b (platelet antigen 2 i.e. PLA2), and 26% are heterozygous (reference 2). Although PTP is a rare condition (approximately 200 reported cases worldwide), NAITP occurs relatively frequently at a rate of 1 in 2000 live births, with about 50% of the cases occurring in first pregnancy (references 2,3) In these conditions, anti-HPA1a antibodies (i.e. type 1a antibodies) attack platelets carrying the HPA1a antigen, leading to destruction of platelets in the patient's blood. In the case of NAITP this manifests itself as a baby with increased tendency to bleeding in view of its reduced platelet count or reduced platelet activity, and consequently impaired blood clotting system.

The early detection of anti-HPA1a antibodies may be of crucial importance as prompt diagnosis may help management of patients, particularly the typing of mother-to-be. There are several methods for the detection of anti-HPA1a antibodies including platelet suspension immunofluorescence test (PSIFT), monoclonal antibody immobilisation of platelet antigens (MAIPA), flow cytometry, and immunosorbent assays (references 4, 5, 6, 7). Although such methods are well established and, in the case if MAIPA, very sensitive, they are time consuming and not amenable to automation and wide-scale application.

Ogden (WO89/02079) and Newman et al (J.Cell Biol (1981) 90 p249-253) describe immunoassays for the detection of antibodies to human platelet antigens, using platelet lysates which are bound to a solid matrix.

Pytela et al (reference 8) describe the binding properties of the platelet membrane glycoprotein GP IIb/IIIa to peptides including the amino acid sequence Arg-Gly-Asp (RGD). The glycoprotein was purified by affinity chromatography of a platelet extract using the heptapeptide GRGDSPK coupled with Sepharose^{RTM}.

Kirchhoffer et al (reference 9) used a similar purification procedure to produce pure GP IIb/IIIa. The pure glycoprotein transferred onto nitrocellulose filters reacted with monocloanal anti-IIb antibody to form an immune complex which was revealed using an enzyme labelled second antibody. The eluate from the affinity chromatography comprises the hexapeptide coupled to the glycoprotein, and may also include Mg²⁺ and Mn²⁺ ions. The presence of the peptide and the metal ions may interfere with use of the glycoprotein in any test for anti-HPA antibodies. Moreover, Kirchhoffer does not suggest the use of genotyped platelets.

R.Kekomaki et al (J.Clin. Invest., 88, 847-854, 1991) (Reference 10) purified GP IIb/IIIa by adsorption onto Sepharose^{RTM} to achieve a rough separation, followed by removal of remaining contaminents by passage over a heparin-Sepharose^{RTM} column, gel filtration with Sephacryl^{RTM} S-300, and removal of trace amounts of fibrinogen by wheat germ agglutinin affinity chromatography.

However, these references do not disclose an immunoassay suitable for the detection of HPA antibodies according to specific genotypes.

### Summary of the Invention

The present invention in one aspect provides an immunoassay for the immunological detection of an antibody to human platelet antigen 1 and/or 3 (HPA1 and/or HPA3) which comprises a solid substrate having coated thereon purified genotyped glycoprotein antigen GPIIIa of genotype HPA1a1a or HPA1b1b, and/or GPIIb of genotype HPA3a3a or HPA3b3b, wherein the glycoprotein(s) is substantially free of peptide including an RGD sequence and of Mg²⁺ and/or Mn²⁺ ions.

The present invention in a further aspect provides an immunoassay for the immunological detection of an antibody to human platelet antigen 1a and/or 3a (HPA1a and/or HPA3a) which comprises a solid substrate having coated thereon purified genotyped glycoprotein antigen GPIIb/IIIa of genotype HPA1a1a/3a3a, HP1a1a/3b3b and/or HPA1b1b/3a3a, wherein the glycoprotein(s) is substantially free of peptide including an RGD sequence and of Mg²⁺ and/or Mn²⁺ ions.

The present invention thus relates to an immunoassay for the immunological detection of an antibody to human platelet antigen 1, 3, 1a, 1b, 3a, or 3b (HPA1, HPA3, HPA1a, HPA1b, HPA3a, or HPA3b) which comprises a solid substrate having coated thereon purified genotyped glycoprotein antigen GPIIIa of genotype HPA1a1a or HPA1b1b, GPIIb of genotype HPA3a3a or HPA3b3b, and/or GPIIb/IIIa of genotype HPA1a1a/3a3a, HPA1a1a/3b3b and/or HPA1b1b/3a3a, wherein the glycoprotein(s) is substantially free of peptide including an RGD sequence and of Mg²⁺ and/or Mn²⁺ ions.

Thus it has been found that anti-HPA1 and anti-HPA3 antibodies play a significant role, particularly in NAITP.

Genotyping of platelets was carried out using the techniques described in Williamson, et al (1992) (Reference 11). Previously it has been found that the antigenic allelic differences (HPA-1-4) are due to polymorphisms contained in the DNA encoding the platelet glycoprotein.

It is possible to type the platelets of a donor or patient using molecular techniques. This is achieved by amplifying extracted genomic DNA using the polymerase chain reaction (PCR) and then subjecting it to restriction fragment length polymorphism (RFLP) analysis. In this way platelets containing the HPA1a, HPA1b, HPA3a or HPA3b or HPA3 antigen can be identified and isolated.

In another aspect, the invention provides an immunoassay process for the in vitro detection of antibodies specific to human platelet antigen 1a, 1b and/or 3a, 3b (HPA1a, HPA1b and/or HPA3a, HPA3b) in serum which comprises
- coating a microtitre plate with purified genotyped glycoprotein antigen GPIIIa of genotype HPA1a1a and/or HPA1b1b and/or GPIIb of genotype HPA3a3a and/or HPA3b3b, wherein the glycoprotein(s) is substantially free of peptide including an RGD sequence and of Mg²⁺ and/or Mn²⁺ ions;
- blocking non-specifically;
- contacting with serum to be tested; and
- detecting the presence of any immune complex formed between the immobilised glycoprotein antigen and any anti-HPA1 or anti-HPA3 antibodies present in the serum.

Generally, the immune complex is estimated by means of anti-human IgG or IgM labelled with an appropriate label, such as an enzyme label (e.g. horseradish peroxidase HRP), a radiolabel, a fluorescence label or other labelling system known in the art. Using an enzyme label, an enzyme-linked immunosorbent assay (ELISA) may be provided which is sensitive, specific, cheap, quick and could potentially be used in large scale screening for anti-HPA1 antibodies. Advantageously, it has been found that the incubations involved in the production of the immune complex, and the estimation thereof with labelled antibody can be carried out at room temperature and so do not require the use of an incubator.

The invention is based on the use of substantially pure human platelet antigen in order to detect the required HPA antibodies. The antigenic determinant for HPA1 is present on the GPIIIa subunit, so that any immunoassay for anti-HPA1 antibodies must include the HPA1 antigen of the glycoprotein GPIIIa subunit. Anti-HPA1 antibodies also exist as types 1a and 1b so that in order to detect anti-HPA1a antibodies, antigen HPA1a of glycoprotein GPIIIa subunit must be selected. It is found that about 98% of the incidence of NAITP is due to the presence of anti-HPA1a or anti-HPA3a antibodies, the prevalence of the former being about four times the latter. Thus, a definitive test for NAITP should also include anti-HPA3a antibodies (HPA3 analogously exists as antigens 3a and 3b). The antigenic determinant for HPA3 lies on the GPIIb subunit, which should therefor be included in any immunoassay for anti-HPA3a antibodies.

In order to assess whether the antibodies are directed to HPA1a or HPA1b (or HPA3a or HPA3b), the appropriate antigen or antigen mixture may be provided in separate wells of the microtitre plate e.g. HPA1a1a/3a3a, HPA1b1b/3b3b, HPA1a1a/3b3b or HPA1b1b/3a3a (each purified from respective genotyped apheresed platelets).

A practical embodiment of the invention as defined in the claims comprises a multiwell microtitre plate for enzyme linked immunoassay (ELISA) having a well containing HPA1a1a/3b3b antigen (to detect anti-HPA1a antibodies) and a well separately containing HPA1b1b/3a3a antigen (to detect anti-HPA3a antibodies); and optionally a well without antigen to act as a control. Typically, each well contains 0.05 to 0.5 micrograms per well; preferably 0.1 micrograms, of glycoprotein.

The wells are generally blocked with a non-specific protein mixture, such as bovine serum albumin prior to contact with blood serum.

There is also described a process for the purification of human platelet antigen glycoprotein GPIIb and/or GPIIIa which comprises;
(a) providing genotyped human platelets
(b) lysing the platelets to form a lysate
(c) applying the lysate to an affinity chromatography substrate having coupled thereto a peptide including the sequence Arg-Gly-Asp (RGD); and
(d) eluting genotyped glycoprotein.

### DETAILED DESCRIPTION OF PREFERRED EMBODIMENTS

Examples of the present invention will now be described by way of example only, with reference to the figures which show;
Figure 1 shows the binding of anti-GPIIIa, IIb, and Ib monoclonal antibodies (MAb) (50, 250 and 1,000 ng/ml respectively) to various amounts of coated HPA.;
Figure 2 shows the sensitivity of the HPA1a ELISA using two anti-HPA1a sera; and
Figure 3 shows a practical example of HPA1a ELISA as a diagnostic test.

### EXAMPLE 1 (Extraction of GP IIb/IIIa)

Reagents were of the Analar grade and were purchased from BDH Chemicals Ltd. (UK) or Aldrich Chemicals Ltd (UK). Anti-GPIIb and IIb/IIIa monoclonal antibodies (MAb) were provided by the Institute for Clinical Immunology and Transfusion Medicine, Justus Liebeg University, Giessen. Anti-GPIb and IIIa MAb were purchased from Dakopatts (Denmark). HRP-conjugated sheep anti-human IgG and IgM were obtained from the Scottish Antibody Production Unit, while HRP-conjugated goat anti-mouse IgG and IgM were purchased from Bio-Rad (UK). Anti-HPA1a and normal sera were obtained from mothers of babies with NAITP and volunteer donors from our centre respectively.

The extraction and purification of GP IIb/IIIa was based on the methods of Pytela et al and Kirchhoffer et al with few modifications (references 8,9). Briefly, a unit of genotyped apheresed platelets (obtained from the Cambridge Transfusion Centre) was washed and the platelets extracted with 10 ml of 100 mM octyl glucopyranoside (OG) and 2mM phenylmethylsulphony fluoride (Sigma, UK) in buffer containing 50 mM Tris/Hcl and 150 mM NaCl, pH 7.5 (TBS). The extract was then centrifuged and the supernatant supplemented with cations (reference 9) and mixed end-over-end with 2 gm of immobilised heptapeptide of sequence GRGDSPK (peptide from Novabiochem, UK; and CNBr activated Sepharose^{RTM} 4B from Pharmacia) at 12 mg peptide/g Sepharose^{RTM} 4B, overnight at 4°C. The beads were then centrifuged in a bench centrifuge (MSE, UK) at 1000 rpm for 5 min at room temperature (RT), and the supernatant removed. The beads were washed by centrifugation until absorbence at 280 nm fell to < 0.05 (Pye Unicam, Philips, UK) indicating that non-specifically bound protein impurity had been washed off. The glycoprotein binds to the heptapeptide immobilised on the Sepharose^{RTM} beads. Then the bound GP IIb/IIIa was eluted by mixing the beads thrice with 5 ml of 1 mM MgCl₂ and 1 mM MnCl₂ in TBS end-over-end for 15 min at room temperature, with the first mixing buffer containing 1 mg/ml GRGDSP hexapeptide (Novabiochem, UK). The use of hexapeptide in the elution buffer competes with the immobilised peptide and liberates a hexapeptide-glycoprotein complex. The elution was repeated three times. The three eluate supernatants, harvested by centrifugation as above, were pooled and centrifuged once more to remove residual bead particles. After recording the absorbence of the eluate, it was dialysed twice at 4°C against 1L of 10 mM Tris/HCl, 150 mM NaCl, pH 7.4. Dialysis is effective to remove the peptide and residual metal ions, and to leave free glycoprotein. Peptide molecules pass through the dialysis membrane and disturb the formation/dissociation equilibrium of the complex in favour of dissociation to the free GP IIb/IIIa. Thus, the glycoprotein will be substantially free of peptide and free of metal ions. A monoclonal antibody to GP IIb/IIIa complex purchased from Binding Site Ltd., Birmingham, UK, bound with greater affinity to non-dialysed GP IIb/IIIa than to dialysed glycoprotein. This indicates that the glycoprotein was substantially free of peptide and metal ions, thus, largely non-complexed. Finally, the absorbence of the dialysed eluate was noted and it was stored in aliquots at -40°C. The immobilised RGD peptide was washed and stored at 4°C for re-use. The protein concentration and purity of the GP IIb/IIIa was assessed by the bicinchoninic assay (Pierce, UK) and SDA-PAGE (silver stain) respectively as before (10). Up to 1.4 mg of GP IIb/IIIa could be obtained from one unit of apheresed platelet concentrate with over 90% purity.

### EXAMPLE 2 (Model ELISA)

The ELISA consisted of the following steps: microtitre plates (Greiner, Germany) were coated with 1, 2 or 3 µg/ml (100µl per well) of one of the following genotyped IIb/IIIa glycoproteins: HPA1a1a/3a3a, HPA1b1b/3b3b, HPA1a1a/3b3b, or HPA1b1b/3a3a (coating buffer only included also for background) overnight at 37°C (ELISA coating, washing, and colour development buffers, and washing procedures were as in reference 12). The wells were washed and blocked with 5% bovine serum albumin (Boseral; Organon Technika) in coating buffer (300 µl/well) for 1 hour at 37°C. After washing the plates were incubated with dilutions of human sera or mouse MAbs at 100 µl/well in triplicate for 1 hour at 37°C. The wells were washed again and incubated with HRP-conjugated anti-human IgG or IgM, or anti-mouse IgG or IgM respectively for 1 hour at 37°C. Alternatively, all incubations can be carried out at room temperature. Finally, the plates were washed and colour developed using tetramethyl benzidine as colourimetric substrate (colour development is enhanced at 37°C). Specific absorbence at 450 nm was equal to the absorbence in GP coated wells minus the absorbence in wells with coating buffer only. Human test and control samples were screened using serum dilutions of 1 in 5.

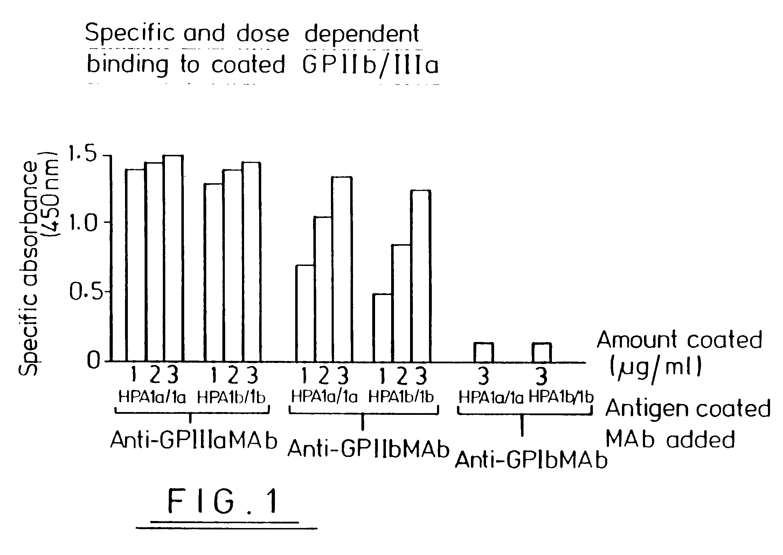

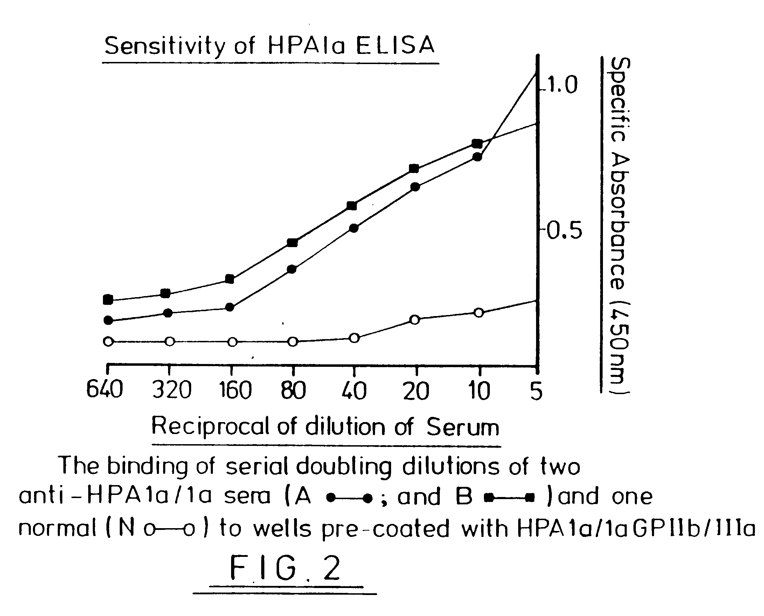

There was a specific and dose dependent binding of coated GP IIb/IIIa to corresponding MAbs (Figure 1) which demonstrates the presence of GPIIIa and GPIIb. For comparison anti-GPIb at relatively high concentrations did not bind to the highest amount of coated GP IIb/IIIa. Specific binding was also observed with anti-GP IIb/IIIa MAb (data not shown). Furthermore, using two human anti-HPA1a positive sera (with normal serum as negative control) the assay was found to be highly sensitive at dilutions of 1:80 to 1:160 (Figure 2). In fact the assay has been found to be capable of being carried out at dilutions of positive sera of 1:1000 to 1:3000. The specificity of the assay was further demonstrated when anti-HPA1a antisera bound only to HPA1a1a/3a3a and HPA1a1a/3b3b, and not to HPA1b1b/3a3a and HPA1b1b/3b3b (data not shown).

### EXAMPLE 3 (Shelflife)

Microtitre plates coated with HPA1a1a/3a3a or HPA1b1b/3b3b overnight, washed, air-dried, and sealed were left at 4°C. Assays carried out weekly thereafter up to 12 weeks using these plates showed consistent and specific binding of two anti-HPA1a sera, but not normal serum (data not shown). Further results to date indicate that plates stored up to 11 months at -20°C perform similarly, and the assessment of coated plates stored at 4°C and -20°C is continuing.

### EXAMPLE 4 (Detection of anti-HPA-1 in human serum)

The suitability of the ELISA for the screening of human anti-HPA1a sera was assessed using sera from mothers of babies with NAITP. Thirty-one sera positive for anti-HPA1a antibodies, two and eight sera which were negative and weak respectively for anti-HPA1a, and four sera whose antibody status was unknown (all determined by PSIFT and MAIPA) were provided by CLB, Amsterdam, and tested blind in the ELISA. Subsequent analysis of the results showed 29/31 agreement of positive sera; 6/8 agreement of weak sera, the other two being negative in the ELISA; the two negative sera were positive in the ELISA; and of the 4 unclear sera, two were weakly positive and two reacted with uncoated wells (similar correlations were observed when coated plates were sent to CLB for ELISA). Subsequent results have shown a complete correlation between the ELISA and the Capture-P assay (Immucor Inc, USA) which is a haemagglutination assay using treated red blood cells.

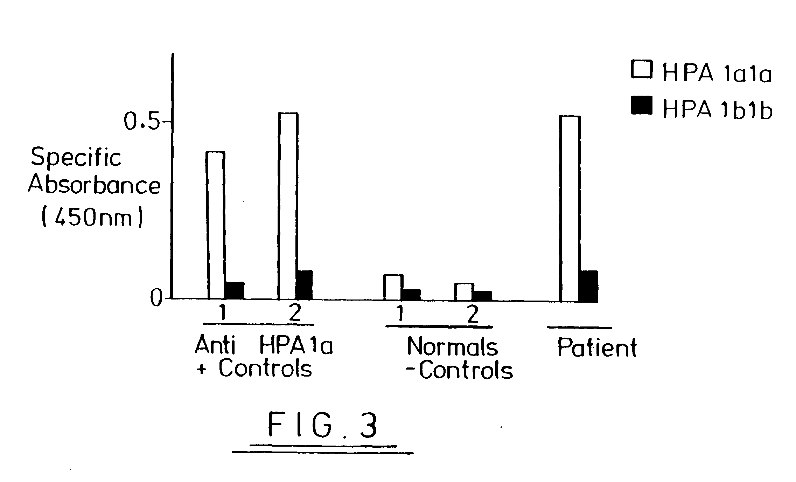

A further benefit of the ELISA is shown in Figure 3. A sixty year old female patient who had undergone a coronary bypass followed by platelet transfusion suffered a severe thrombocytopenia about a week later. Since she was receiving heparin, it was important to determine the cause of the thrombocytopenia. Using the ELISA we were able to establish within 3.5 hours of receiving her serum sample that it contained strong anti-HPA1a antibody.

Our results show that the newly developed ELISA is a simple, cheap, quick, specific and sensitive assay. In exceptional cases where high non-specific reactivity occurs with uncoated wells, higher dilutions may be used or confirmation may be sought using another assay. However, the stability of coated plates, the potential of developing the ELISA for other HPA antibodies, and its amenability for automation should enable the ELISA to be used widely for the screening of anti-platelet antibodies, including large scale population studies in NAITP. Furthermore, the observation of high correlation between PSIFT/MAIPA and the ELISA indicates that the ELISA could be developed as a diagnostic test. Finally, it is possible that the ELISA, due to its enhanced sensitivity and accessibility, may detect antibodies missed by other assays.

### REFERENCES

1. PUIG, N., SAYAS, M.J., MONTORO, J.A., VILLALBA, J.V. AND PLA, A. Post-transfusion purpura as the main manifestation of transfusion reaction, responsive to steroids: flow-cytometric investigation of granulocyte and platelet antibodies. Ann Hemat., 62. 232-234 (1991).
2. NEWMAN, P. Platelet GPIIb-IIIa: Molecular variations and alloantigens. Thromb. Haem., 66, 111-118 (1991).
3. KAPLAN, C., DAFFOS, F., FORESTIER, F., MOREL, M.C., CHESNEL, N., AND TCHERNIA, G. Current trends in neonatal allimmune thrombocytopenia: Diagnosis and therapy. In, platelet immunology fundamental and clinical aspects. Eds. Kaplan-Gouet, C., Schlegel, N., Salmon, Ch., McGregor, J., Inserm/John Libbey Eurotext Ltd., 206, 267-278 (1991).
4. BORNE, D.E.G. KR VON DEM, VER HEUGHT, F.W.A., OOSTERHOF, F., RIESZ, E. VON, BRUTEL DE LA RIVIERE, A., AND ENGELFRIET, C.P. A simple immunofluorescence test for the detection of platelet antibodies. Br. J. Haem., 39, 195-207 (1978).
5. KIEFEL, V. The MAIPA assay and its applications in immunohaematology. Transfusion Med., 2, 181-188 (1992).
6. FREEDMAN, J., AND HORNSTEIN, A. Simple method for differentiating between HLA and platelet specific antibodies by flow cytometry. Am. J. Haem., 38, 314-320 (1991).
7. BORZINI, P. ELISA procedures for the characterisation of platelet specific antibodies. Evaluation of some variables. Haematologia, 76, 20-27 (1991).
8. PYTELA, R., PIERSCHBACHER, M.D., GINSBERG, M.H., PLOW, E.F., AND RUOSLAHTI, E. Platelet membrane glycoprotein IIb/IIIa: member of a family of Arg-Gly-Asp-specific adhesion receptors. Science, 231, 1559-1561 (1986).
9. KIRCHHOFFER, O., GAILIT, J., RUOSLAHTI, E., GRZESIAK, J., PIERSCHBACHER, M.D. Cation-dependent Changes in the Binding Specificity of the Platelet Receptor GP IIb/IIIa Journal of Biol.Chem 265 p18525-18530 (1990).
10. KEKOMAKI, R., DAWSON, B., McFARLAND, J., and KUNICKI, T.J. Localisation of Human Platelet Auto antigens to the Cysteine-rich region of glycoprotein IIIa J.Clin. Invest. 88 p847-854 (1991).
11. WILLIAMSON, L.M., BRUCE, D., LUBENKO, A., CHANA, H.J., and OUWEHAND, W.H. Molecular biology for platelet alloantigen typing Transfusion medicine 2 p255-264 (1992).
12. BESSOS, H., AND MURPHY, W.G. A new competitive binding enzyme-linked immunosorbent assay for glycocalicin in plasma and platelet concentrate supernatants. Thromb. Res., 59, 497-507 (1990).

## Claims

1. An immunoassay for the immunological detection of an antibody to human platelet antigen 1 and/or 3 (HPA1 and/or HPA3) which comprises a solid substrate having coated thereon purified genotyped glycoprotein antigen GPIIIa of genotype HPA1a1a or HPA1b1b, and/or GPIIb of genotype HPA3a3a or HPA3b3b, wherein the glycoprotein(s) is substantially free of peptide including an RGD sequence and of Mg²⁺ and/or Mn²⁺ ions.

2. An immunoassay according to claim 1 wherein the substrate is coated with purified genotyped glycoprotein antigen GPIIb/IIIa of genotype HPA1a1a/3a3a, HPA1a1a/3b3b, HPA1b1b/3a3a and/or HPA1b1b/3b3b.

3. An immunoassay for the immunological detection of an antibody to human platelet antigen 1a and/or 3a (HPA1a and/or HPA3a) which comprises a solid substrate having coated thereon purified genotyped glycoprotein antigen GPIIb/IIIa of genotype HPA1a1a/3a3a, HPA1a1a/3b3b and/or HPA1b1b/3a3a, wherein the glycoprotein(s) is substantially free of peptide including an RGD sequence and of Mg²⁺ and/or Mn²⁺ ions.

4. An immunoassay according to any preceding claim wherein the glycoprotein antigen has been purified by batch affinity chromatography on an affinity substrate having coupled thereto a peptide including the sequence Arg-Gly-Asp (RGD).

5. An immunoassay according to claim 4 wherein the peptide has the sequence Gly-Arg-Gly-Asp-Ser-Pro-Lys (GRGDSPK).

6. An immunoassay according to any preceding claim wherein the solid substrate is a multiwell microtitre plate.

7. An immunoassay according to claim 6 for carrying out an enzyme linked immunoassay, which further comprises;
- a non-specific blocking agent;
- an anti-human immunoglobulin labelled with an enzyme label; and
- a substrate capable of giving a colourimetric reaction with said enzyme label.

8. An immunoassay according to claim 7 which comprises a well coated with HPA1a1a/3b3b glycoprotein to detect anti-HPA1a antibodies; and a well coated with HPA1b1b/3a3a glycoprotein to detect anti-HPA3a antibodies.

9. An immunoassay according to any preceding claim wherein the genotyped glycoprotein antigen GPIIb and/or GPIIIa has been purified according to the following process of:
(a) providing genotyped human platelets
(b) lysing the platelets to form a lysate
(c) applying the lysate to an affinity chromatography substrate having coupled thereto a peptide including the sequence Arg-Gly-Asp (RGD); and
(d) eluting genotyped glycoprotein.

10. An immunoassay according to claim 9 wherein the peptide coupled to the affinity substrate is Gly-Arg-Gly-Asp-Ser-Pro-Lys (GRGDSPK).

11. An immunoassay according to either of claims 9 or 10 wherein the glycoprotein is eluted using an elution medium containing a peptide including the sequence RGD.

12. An immunoassay according to any one of claims 9 to 11 wherein the eluted glycoprotein containing peptide coupled thereto is dialysed to remove peptide and generate glycoprotein without coupled peptide.

13. An immunoassay process for the in vitro detection of antibodies specific to human platelet antigen 1a, 1b and/or 3a, 3b (HPA1a, HPA1b and/or HPA3a, HPA3b) in serum which comprises
- coating a microtitre plate with purified genotyped glycoprotein antigen GPIIIa of genotype HPA1a1a and/or HPA1b1b and or GPIIb of genotype HPA3a3a and/or HPA3b3b, wherein the glycoprotein(s) is substantially free of peptide including an RGD sequence and of Mg²⁺ and/or Mn²⁺ ions.
- blocking non-specifically;
- contacting with serum to be tested; and
- detecting the presence of any immune complex formed between the immobilised glycoprotein antigen and any anti-HPA1 or anti-HPA3 antibodies present in the serum.

## Patentansprüche

1. Immunassay für den immunologischen Nachweis eines Antikörpers für Humanthrombozytenantigen 1 und/oder 3 (HPA1 und/oder HPA3), der umfaßt ein festes Substrat mit darauf überzogenem gereinigtern genotypisierten Glycoproteinantigen GPIIIa vom Genotyp HPA1a1a oder HPA1b1b, und/oder GPIIb vom Genotyp HPA3a3a oder HPA3b3b, wobei das (die) Glycoprotein(e) im wesentlichen frei ist (sind) von Peptid, einschließend eine RGD Sequenz, und von Mg²⁺ und/oder Mn²⁺ Ionen.

2. Immunassay nach Anspruch 1, wobei das Substrat mit gereinigtem genotypisierten Glycoproteinantigen GPIIb/IIIa vom Genotyp HPA1a1a/3a3a, HPA1a1a/3b3b, HPA1b1b/3a3a und/oder HPA1b1b/3b3b überzogen ist.

3. Immunassay für den immunologischen Nachweis eines Antikörpers für Humanthrombozytenantigen 1a und/oder 3a (HPA1a und/oder HPA3a), der umfaßt ein festes Substrat mit darauf überzogenem gereinigten genotypisierten Glycoproteinantigen GPIIb/IIIa vom Genotyp HPA1a1a/3a3a, HPA1a1a/3b3b und/oder HPA1b1b/3a3a, wobei das (die) Glycoprotein(e) im wesentlichen frei ist (sind) von Peptid, einschließend eine RGD Sequenz, und von Mg²⁺ und/oder Mn²⁺ Ionen.

4. Immunassay nach einem vorhergehenden Anspruch, wobei das Glycoproteinantigen gereinigt worden ist durch Chargenaffinitätschromatographie auf einem Affinitätssubstrat, mit einem daran gekoppelten Peptid, einschließend die Sequenz Arg-Gly-Asp (RGD).

5. Immunassay nach Anspruch 4, wobei das Peptid die Sequenz Gly-Arg-Gly-Asp-Ser-Pro-Lys (GRGDSPK) hat.

6. Immunassay nach einem vorhergehenden Anspruch, wobei das feste Substrat eine Multivertiefungsmikrotiterplatte ist.

7. Immunassay nach Anspruch 6 zum Durchführen eines Enzym gekoppelten Immunassays, der ferner umfaßt
- ein nicht spezifisches Blockierungsmittel,
- ein Anti-Human-Immunglobulin, markiert mit einer Enzymmarkierung, und
- ein Substrat, fähig zum Geben einer kolorimetrischen Reaktion mit der Enzymmarkierung.

8. Immunassay nach Anspruch 7, der umfaßt eine Vertiefung, überzogen mit HPA1a1a/3b3b Glycoprotein zum Nachweisen von Anti-HPA1a Antikörpern, und eine Vertiefung, überzogen mit HPA1b1b/3a3a Glycoprotein zum Nachweisen von Anti-HPA3a Antikörpern.

9. Immunassay nach einem vorhergehenden Anspruch, wobei das genotypisierte Glycoproteinantigen GPIIb und/oder GPIIIa gemäß dem folgenden Verfahren gereinigt worden ist:
(a) zur Verfügung stellen von genotypisierten Humanthrombozyten,
(b) Lysieren der Thrombozyten unter Bilden eines Lysats,
(c) Anwenden des Lysats auf ein Affinitätschromatographiesubstrat mit einem daran gekoppelten Peptid, einschließend die Sequenz Arg-Gly-Asp (RGD), und
(d) Eluieren von genotypisiertem Glycoprotein.

10. Immunassay nach Anspruch 9, wobei das an das Affinitätssubstrat gekoppelte Peptid Gly-Arg-Gly-Asp-Ser-Pro-Lys (GRGDSPK) ist.

11. Immunassay nach entweder Anspruch 9 oder 10, wobei das Glycoprotein eluiert wird unter Verwenden eines Elutionsmediums, enthaltend ein Peptid, einschließend die Sequenz RGD.

12. Immunassay nach einem der Ansprüche 9 bis 11, wobei das eluierte Glycoprotein, enthaltend daran gekoppeltes Peptid, dialysiert wird unter Entfernen von Peptid und Erzeugen von Glycoprotein ohne gekoppeltes Peptid.

13. Immunassayverfahren für den in vitro Nachweis von Antikörpern, spezifisch für Humanthrombozytenantigen 1a, 1b und/oder 3a, 3b (HPA1a, HPA1b und/oder HPA3a, HPA3b) in Serum, der umfaßt
- Übeziehen eine Mikrotiterplatte mit gereinigtem genotypisiertem Glycoproteinantigen GPIIIa vom Genotyp HPA1a1a und/oder HPA1b1b und/oder GPIIb vom Genotyp HPA3a3a und/oder HPA3b3b, wobei das (die) Glycoprotein(e) im wesentlich frei ist von Peptid, einschließend eine RGD Sequenz, und von Mg²⁺ und/oder Mn²⁺ Ionen,
- nicht spezifisch blockieren,
- in Kontakt bringen mit zu testendem Serum und
- Nachweisen des Vorhandenseins irgendeines Immunkomplexes, gebildet zwischen dem immobolisierten Glycoproteinantigen und irgendeinem Anti-HPA1 oder Anti-HPA3 Antikörpern, vorhanden in dem Serum.

## Revendications

1. Immunoanalyse pour la détection immunologique d'un anticorps dirigé contre l'antigène plaquettaire humain 1 et/ou 3 (HPA1 et/ou HPA3) qui comprend un substrat solide qui a été revêtu d'un antigène de glycoprotéine génotypée purifié GPIIIa de génotype HPA1a1a ou HPA1b1b, et/ou GPIIb de génotype HPA3a3a ou HPA3b3b, où la ou les glycoprotéines sont pratiquement exemptes de peptide comprenant une séquence RGD et d'ions Mg²⁺ et/ou Mn²⁺.

2. Immunoanalyse selon la revendication 1 dans laquelle le substrat est revêtu d'un antigène de glycoprotéine génotypée purifié GPIIb/IIIa de génotype HPA1a1a/3a3a, HPA1a1a/3b3b, HPA1b1b/3a3a et/ou HPA1b1b/3b3b.

3. Immunoanalyse pour la détection immunologique d'un anticorps dirigé contre l'antigène plaquettaire humain 1a et/ou 3a (HPA1a et/ou HPA3a) qui comprend un substrat solide qui a été revêtu d'un antigène de glycoprotéine génotypée purifié GPIIb/IIIa de génotype HPA1a1a/3a3a, HPA1a1a/3b3b et/ou HPA1b1b/3a3a, où la ou les glycoprotéines sont pratiquement exemptes de peptide comprenant une séquence RGD et d'ions Mg²⁺ et/ou Mn²⁺.

4. Immunoanalyse selon l'une quelconque des revendications précédentes dans laquelle l'antigène de glycoprotéine a été purifié par chromatographie d'affinité discontinue sur un substrat d'affinité auquel était couplé un peptide comprenant la séquence Arg-Gly-Asp (RGD).

5. Immunoanalyse selon la revendication 4 dans laquelle le peptide a la séquence Gly-Arg-Gly-Asp-Ser-Pro-Lys (GRGDSPK).

6. Immunoanalyse selon l'une quelconque des revendications précédentes dans laquelle le substrat solide est une plaque de microtitrage multipuits.

7. Immunoanalyse selon la revendication 6 pour réaliser un immunodosage enzymatique, qui comprend en outre;
- un agent bloquant non spécifique;
- un anti-immunoglobulines humaines marqué avec un marqueur enzymatique; et
- un substrat capable de donner une réaction colorimétrique avec ledit marqueur enzymatique.

8. Immunoanalyse selon la revendication 7 qui comprend un puits revêtu d'une glycoprotéine HPA1a1a/3b3b pour détecter des anticorps anti-HPA1a; et un puits revêtu d'une glycoprotéine HPA1b1b/3a3a pour détecter des anticorps anti-HPA3a.

9. Immunoanalyse selon l'une quelconque des revendications précédentes dans laquelle l'antigène de glycoprotéine génotypée GPIIb et/ou GPIIIa a été purifié conformément au procédé suivant consistant à:
(a) fournir des plaquettes humaines génotypées
(b) lyser les plaquettes pour former un lysat
(c) déposer le lysat sur un substrat de chromatographie d'affinité auquel a été couplé un peptide comprenant la séquence Arg-Gly-Asp (RGD); et
(d) éluer la glycoprotéine génotypée.

10. Immunoanalyse selon la revendication 9 dans laquelle le peptide couplé au substrat d'affinité est Gly-Arg-Gly-Asp-Ser-Pro-Lys (GRGDSPK).

11. Immunoanalyse selon l'une ou l'autre des revendications 9 ou 10 dans laquelle la glycoprotéine est éluée au moyen d'un milieu d'élution contenant un peptide comprenant la séquence RGD.

12. Immunoanalyse selon l'une quelconque des revendications 9 à 11 dans laquelle la glycoprotéine éluée contenant le peptide couplé à celle-ci est dialysée pour éliminer le peptide et produire une glycoprotéine sans peptide couplé.

13. Procédé d'immunoanalyse pour la détection in vitro d'anticorps spécifiques de l'antigène plaquettaire humain 1a, 1b et/ou 3a, 3b (HPA1a, HPA1b et/ou HPA3a, HPA3b) dans un sérum qui comporte les étapes de:
- revêtement d'une plaque de microtitrage avec un antigène de glycoprotéine génotypée purifiée GPIIIa de génotype HPA1a1a et/ou HPA1b1b et/ou GPIIb de génotype HPA3a3a et/ou HPA3b3b, où la ou les glycoprotéines sont pratiquement exemptes de peptide comprenant une séquence RGD et d'ions Mg²⁺ et/ou Mn²⁺;
- blocage non spécifique;
- mise en contact avec le sérum à tester; et
- détection de la présence de tout immuncomplexe formé entre l'antigène de glycoprotéine immobilisé et les anticorps anti-HPA1 ou anti-HPA3 éventuels présents dans le sérum.
